**Europäisches Patentamt**

(19) **European Patent Office**

**Office européen des brevets**

(11) Veröffentlichungsnummer: **0 093 887**
**B1**

(12) EUROPÄISCHE PATENTSCHRIFT

(45) Veröffentlichungstag der Patentschrift:
08.01.86

(51) Int. Cl.⁴: **A 61 M 25/00,** A 61 M 1/14

(21) Anmeldenummer: **83103584.5**

(22) Anmeldetag: **14.04.83**

(54) **Perfusionskatheter.**

(30) Priorität: **20.04.82 DE 3214397**

(43) Veröffentlichungstag der Anmeldung:
**16.11.83 Patentblatt 83/46**

(45) Bekanntmachung des Hinweises auf die Patenterteilung:
**08.01.86 Patentblatt 86/2**

(84) Benannte Vertragsstaaten:
**AT BE CH DE FR GB IT LI LU NL SE**

(56) Entgegenhaltungen:
**EP - A - 0 000 041**
**EP - A - 0 036 642**
**DE - A - 2 703 087**
**US - A - 4 134 402**

(73) Patentinhaber: **Aigner, Karl, Dr., Uhlandstrasse 5,**
**D-6301 Pohlheim (DE)**

(72) Erfinder: **Aigner, Karl, Dr., Uhlandstrasse 5,**
**D-6301 Pohlheim (DE)**

(74) Vertreter: **Sternagel, Hans-Günther, Dr. et al,**
**Patentanwälte Dr. M. Hann Dr. H.-G. Sternagel**
**Marburger Strasse 38, D-6300 Giessen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

## Beschreibung

Gegenstand der Erfindung ist ein Perfusionsdoppelkatheter, der insbesondere zur isolierten Perfusion der Leber im Rahmen einer intraarteriellen Chemotherapie verwendet wird.

Katheter für medizinische Zwecke sind seit langem bekannt und finden vielfach Anwendung.

In DE-A-3010841 ist ein Doppelkatheter beschrieben, dessen Katheterrohre gegeneinander verschiebbar ausgebildet sind und der für die Venenpunktion zur Hämodialyse verwendet werden kann. Durch die Verschiebbarkeit der Einzelkatheter können die Eintrittsöffnungen bzw. Austrittsöffnung der Einzelkatheter an ihren Spitzen nach dem Einführen in ein Blutgefäss in einem solchen Abstand voneinander plaziert werden, dass in das Blutgefäss zurückgeführtes Blut nicht in den Bereich der Entnahmestelle gelangt.

In EP-A-000041 ist ein Doppellumen-Katheter beschrieben, der zwei seitliche Anschlüsse aufweist.

Der aus US-A-4134402 bekannte Doppel-Katheter weist ein längeres Katheterrohr mit einem aufgesetzten kürzeren Katheterrohr auf. Die Querschnitte der Rohre sind gleich gross und das längere Katheterrohr weist eine abgeschrägte vordere Öffnung auf. Das kürzere Katheterrohr weist auf der Oberseite eine Öffnung auf, die durch Verdrehen des doppelwandigen Rohres gegeneinander verschliessbar ist. Diese beiden bekannten Katheter können nur teilweise in ein Blutgefäss eingeführt werden und dienen zur gleichzeitigen Abnahme und Rückführung von Blut.

Aus DE-A-2703087 ist ein doppellumiger Katheter zur extrakorporalen Hämodialyse mit zwei getrennten Kanülen zur Blutentnahme und Rückführung bekannt. Die Katheterrohre sind koaxial zueinander angeordnet. An dem nicht in das Blutgefäss einzuführenden Ende ist ein Anschlussstück vorhanden mit einem seitlichen Anschluss für die Aussenkanüle. Bei der intraarteriellen Chemotherapie lassen sich die bekannten Katheter jedoch nicht in allen Fällen verwenden, insbesondere, wenn es sich um die isolierte Perfusion bestimmter Organe oder Körperteile handelt.

Die hämatogene Metastasierung von Tumoren, vor allem aus dem Gastrointestinalbereich in die Leber erfolgt über die Portalvene. Aus diesem Grunde war es wünschenswert, eine isolierte Perfusion der tumorös besiedelten Leber über die Arteria hepatica und die Vena portae durchzuführen.

Aufgabe der vorliegenden Erfindung ist es, eine konstruktive Gestaltung eines Perfusionskatheters zu schaffen, die eine isolierte Perfusion der Leber ermöglicht, ohne dass der Durchfluss durch die Vena cava und die Vena portae unterbrochen werden müssen, und die ein isoliertes Abziehen des Blutes aus der Leber während der Perfusion ermöglicht.

Diese Aufgabe wird gelöst durch einen als Doppelkatheter ausgebildeten Perfusionskatheter, dessen einzelne fest miteinander verbundene Katheterrohre unterschiedliche Längen, unterschiedliche Querschnitte und unterschiedlich abgeschrägte vordere Öffnungen aufweisen und von dessem Katheterrohr mit grösserem Aussendurchmesser ein Anschluss seitlich abgeführt ist, der dadurch gekennzeichnet ist, dass er aus einem Schienungskatheter mit einem aufgesetzten kürzeren Katheterrohr, dessen hinteres Ende seitlich vom Schienungskatheter abgeführt ist, besteht, wobei am Schienungskatheter in Abstand von seinem hinteren Ende als seitlich abgeführter Anschluss ein Nebenrohr und in einem Abstand von 40 bis 60 mm vom Mittelpunkt der Anschlussstelle des Nebenrohres mindestens eine Öffnung vorhanden sind.

In den abhängigen Ansprüchen sind bevorzugte Ausführungsformen des Katheters beschrieben.

Der erfindungsgemässe Katheter ist als ein doppellumiger Katheter oder als Doppelkatheter ausgebildet. Der grossvolumige Teil dient der Schienung des Hauptgefässes, durch das das Blut aus den unteren Extremitäten und den Nieren zur rechten Herzkammer strömt. Ein aufgesetztes kürzeres zweites Katheterrohr mit engerem Durchmesser dient dazu, das venöse Leberblut während der Perfusion abzuführen und ist in der Nähe des hinteren Endes des Schienungskatheters von diesem abgeführt und wird mittels Schlauchverbindung an eine Herz-Lungen-Maschine angeschlossen. Der Schienungskatheter weist an einer bestimmten Stelle ein oder zwei seitlich angeordnete Löcher auf, durch die während der Perfusion das venöse Blut der Vena renalis in den in der Vena cava angeordneten Schienungskatheter eintreten kann. In bestimmtem Abstand von dieser Öffnung oder diesen Öffnungen, die vorzugsweise oval in Längsrichtung des Katheters ausgebildet sind, weist der Schienungskatheter ein Nebenrohr auf zum Anschluss eines Ultrafiltrationsfilters und einer Pumpe, über die während der Perfusion das aus der gegen die Leber abgeklemmten Vena portae zufliessende Blut in Form eines temporären portocavalen Nebenschlusses in den Schienungskatheter und damit in die Vena cava eingeführt wird. In diesen Nebenschluss ist eine Rollerpumpe und ein Ultrafiltrationsfilter zur partiellen Entgiftung des Pfortaderblutes vorhanden. Der Schienungskatheter weist also eine vordere Öffnung, eine hintere Öffnung, ein oder zwei seitliche Öffnungen und einen Nebenanschluss auf und auf das dickere Schienungskatheterrohr ist ein zweites kürzeres Katheterrohr aufgesetzt, das in unmittelbarer Nähe des Nebenanschlusses seitlich weggeführt ist. Im vorderen Teilstück weist der Doppelkatheter nur ein Rohr auf, im mittleren Teilstück zwei parallel laufende Rohre und am hinteren Ende wieder nur ein Rohr mit dem in Abstand vom hinteren Ende angeordneten Nebenrohr. Das vordere Ende des Schienungskatheters ist abgeschrägt, um die Einführung in das Blutgefäss zu erleichtern. Das Ende des kürzeren Katheterrohres ist abgeschrägt, so dass der Übergang zum Doppelrohr mit einem sich kontinuierlich vergrössernden Aussenumfang des Katheters zum Doppelrohr vollzieht. Es ist von wesentlicher Bedeutung, dass dieser Übergang fliessend und ohne Kanten erfolgt, so dass die Einführung des Doppelrohrteiles in das Gefäss erleichtert ist. Vorzugsweise weist das abgeschrägte Ende des seitlich aufgesetzten kürzeren Katheterrohres ein oder zwei gegebenenfalls seitlich versetzte Öffnungen auf, um eine Stirnkantenbildung zu vermeiden. Der Querschnitt des Doppelkatheterrohres ist oval oder eiförmig. Im vorderen Teil ist der Querschnitt des Schienungskatheterrohres vorzugsweise kreisförmig ausgebildet. Der Innendurchmesser des Schienungskatheters ist vorzugsweise grösser als der Innendurchmesser des kürzeren Katheterrohres. Die seitliche Abführung des kürzeren Katheterrohres vom Schienungskatheter ist in der Nähe der Anschlussstelle des Nebenrohres angeordnet, vorzugsweise in unmittelbarer Nachbarschaft.

Das Schienungskatheterrohr weist in einem Abstand vom Ende des aufgesetzten kürzeren Katheterrohres seitlich ein oder zwei Öffnungen auf, die sich gegenüberliegen können oder an der vom kürzeren Katheterrohr abgewandten Seite in Querrichtung nebeneinander angeordnet sein können. Vorzugsweise sind die Öffnungen oval mit der Längsseite in Längsrichtung des Schienungskatheters ausgebildet. Der Mittelpunkt der Öffnungen im Schienungskatheter weist einen bestimmten Abstand vom Mittelpunkt der Anschlussstelle des Nebenrohres auf. Der Abstand kann 40-60 mm betragen, vorzugsweise ist er 50 mm. Die Länge der ovalen Öffnung liegt zwischen 10 und 20 mm und beträgt vorzugsweise 16 mm.

Um die seitlichen Öffnungen im hinteren Teil des Schienungskatheters während des Einführens in das Organ vorübergehend verschliessen zu können, ist in dem Schienungskatheterrohr ein verschlossenes Schlauchstück oder ein aus vollem Material bestehender Stab vorhanden, dessen Aussendurchmesser etwas geringer ist als der Innendurchmesser des Schienungskatheters. Dieses Teil ist so lang ausgebildet, dass es in eingeschobenem Zustand die seitlichen Löcher verdeckt und noch über das hintere Ende des Katheters hinausragt, so dass ein Herausziehen aus dem Schienungskatheter möglich ist, sobald der Katheter so weit in das Gefäss eingeführt ist, dass die Löcher sich innerhalb des Blutgefässes befinden.

An das Nebenanschlussrohr des Perfusionskatheters ist mittels einer Schlauchverbindung ein Ultrafiltrationsfilter und an dieses die Druckseite einer Pumpe angeschlossen, um auf diesem Wege den temporären portocavalen Nebenschluss der Vena portae mit der Vena cava über den bei der Perfusion in der Vena cava angeordneten Doppelkatheter herzustellen.

Der Perfusionskatheter kann aus den für Kathetermaterialien üblichen und geeigneten Materialien hergestellt werden. Derartige Materialien verhalten sich neutral gegenüber der Körperflüssigkeit, lassen sich ohne Probleme sterilisieren und sind ausreichend elastisch, aber andererseits auch genügend steif und fest, um in Blutgefässe eingeführt zu werden. Geeignete Materialien sind Polyolefine, polyfluorierte Kohlenwasserstoffpolymere, synthetische Kautschuke und dergleichen, ein ganz besonders bevorzugtes Material ist Siliconkautschuk.

Bei der Verwendung wird der Schienungskatheter in seiner gesamten Länge in der Vena cava plaziert. Durch Abbinden des Gefässes von aussen werden die erforderlichen getrennten Räume und die Abdichtung nach aussen hergestellt. Da das Abbinden in der Nähe der Spitze des Schienungskatheters wegen der unmittelbaren Nähe des Herzens in manchen Fällen schwierig ist, weist eine bevorzugte Ausbildungsform des erfindungsgemässen Perfusionskatheters in der Nähe der Spitze des Schienungskatheters einen aussen aufgesetzten aufblasbaren Ballon auf. Durch Aufblasen des Ballons über eine Zuführleitung ist eine Abdichtung innerhalb des Gefässes möglich, so dass das Abbinden von aussen entfällt.

Der Gegenstand der Erfindung wird nun anhand der Abbildungen noch näher beschrieben.

Fig. 1 zeigt einen schematischen Längsschnitt durch das Perfusionskatheterteil, das bei der Perfusion in das Hauptgefäss eingesetzt wird.

Fig. 2 ist ein Querschnitt des Doppelkatheters längs der Linie A-A von Fig. 1 und

Fig. 3 ist ein Querschnitt des Doppelkatheters längs der Linie B-B von Fig. 1, aus der die Lage der seitlichen Öffnungen zu ersehen ist.

Fig. 4 zeigt die Ausführungsform mit aufgesetztem aufblasbaren Ballon.

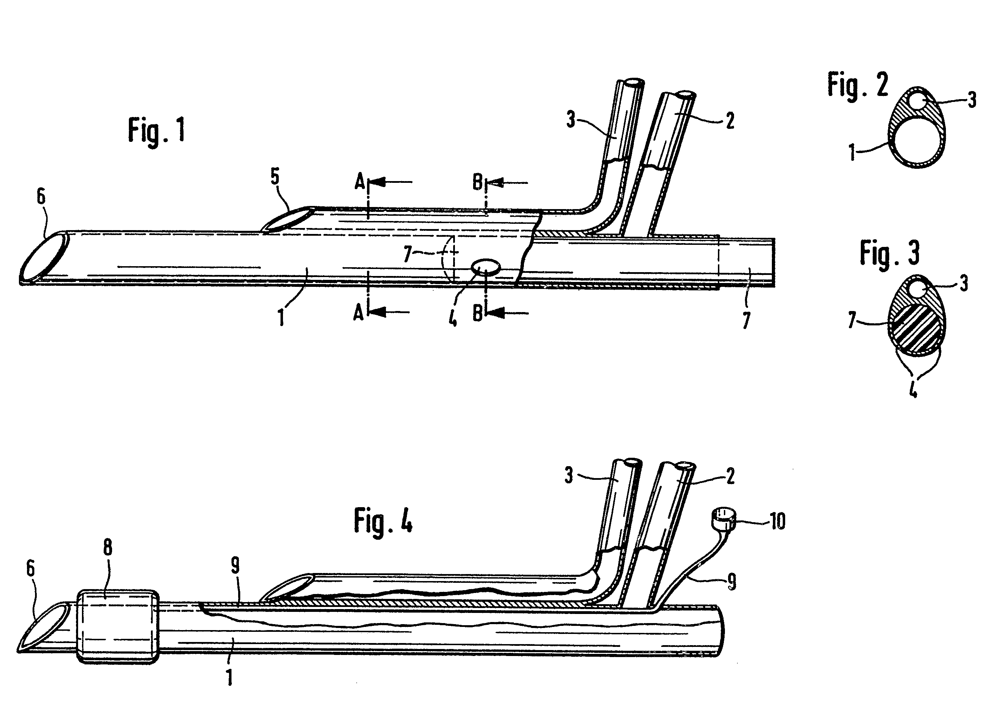

In Fig. 1 ist der Schienungskatheter mit 1 bezeichnet, der mit seinem vorderen Ende 6 in das Gefäss eingeführt wird. Aufgesetzt auf den Schienungskatheter 1 ist das zweite Katheterrohr 3, das kürzer ausgebildet ist und dessen Ende 5 so abgeschrägt ausgebildet ist, dass sich der Aussenumfang des Doppelkatheters in diesem Teilbereich kontinuierlich vergrössert. Das hintere Ende des kürzeren Katheterrohres wird im hinteren Teil des Schienungskatheters in der Nähe des Nebenrohres 2 seitlich abgeführt. Im Abstand von einigen cm vom hinteren Ende des Schienungskatheters 1 ist das Nebenrohr 2 angebracht. In bestimmtem Abstand von dieser Anschlussstelle in Richtung auf sein vorderes Ende weist der Schienungskatheter 1 die seitliche Öffnung 4 auf. Diese Öffnungen sind vorzugsweise in Längsrichtung oval ausgebildet und ist einige mm breit und etwa 10-20 mm, vorzugsweise 16 mm lang. Der Schienungskatheter hat eine Länge von etwa 250 mm, sein Innendurchmesser beträgt 8-16 mm, vorzugsweise 10 mm und die Wandstärke beträgt 1-2 mm. Der Mittelpunkt der Anschlussstelle ist vorzugsweise 20 mm vom hinteren Ende des Katheterrohres entfernt. Das zweite Katheterrohr ist kürzer ausgebildet und das vordere Ende befindet sich etwa 80-120 mm hinter dem vorderen Ende 6 des Schienungskatheters. Der Innendurchmesser des kürzeren Katheterrohres kann 4-6 mm betragen. Der Innendurchmesser des Nebenrohres 2 beträgt 4-6 mm, wobei die Wandstärken zwischen 1 und 2 mm liegen.

In den Schienungskatheter 1 eingeschoben ist vom hinteren Ende her ein geschlossenes Schlauchstück oder ein Vollmaterialstab 7, um das Loch 4 während des Einbringens des Doppelkatheters in das Gefäss verschliessen zu können. Der Aussendurchmesser dieses Teils ist so gewählt, dass er die seitlichen Löcher im eingeschobenen Zustand 4 verschliesst, jedoch leicht aus dem Schienungskatheter 1 herausziehbar ist.

Nicht gezeigt in Fig. 1 sind die an das Nebenrohr 2 angeschlossenen Teile zur Herstellung des temporären portocavalen Nebenschlusses. Dies sind über eine Schlauchverbindung ein angeschlossenes Hämofilter, um eine Ultrafiltration zur partiellen Entgiftung des Pfortaderblutes auszuführen und eine Rollerpumpe zur Förderung der Flüssigkeit. Die Schlauchverbindung zwischen dem Ultrafiltrationsfilter und dem Nebenrohr 2 hat eine Länge von etwa 2,5 m. Zwischen dem Filter und der Schlauchpumpe oder Rollerpumpe ist ein Schauchstück mit einer Gummimembran zur Blutentnahme zwischengeschaltet. Der Schlauchabstand zwischen der Schlauchpumpe und dem Filter beträgt ca. 1,5 m und etwa 3 m Schlauchverbindung sind zum Anschluss der Pumpe an die Vena portae erforderlich. Das Filter ist ein übliches Ultrafiltrationsfilter mit Membranflächen von 0,5-1,5 m² Membranfläche, vorzugsweise 1 m² Membranfläche, wobei die Membranen eine Ausschlussgrenze beim Molekulargewicht von etwa 50 000 aufweisen. Das aus dem eigentlichen Filter abgeführte Ultrafiltrat wird volumenmässig in einem geeichten Messgefäss mit einer Genauigkeit von ±5 ml gemessen und in der Nähe des Filters zwischen dem Filter und dem Nebenrohr 2 ist ein Schlauchanschlussteil vorhanden, um die als Ultrafiltrat abgezogene Flüssigkeitsmenge in Form einer Ersatzlösung in das Schlauchsystem wieder einzuspeisen. Bei den Ersatzlösungen handelt es sich um geeignete Substitutionsflüssigkeiten. Die Schlauchvervindungen haben einen Innendurchmesser von etwa 5 mm und einen Aussendurchmesser von etwa 7 mm und sind aus geeigneten Materialien, vorzugsweise aus synthetischen Kautschuken oder Siliconkautschuk hergestellt.

Fig. 2 zeigt den Doppelkatheter im Querschnitt längs der Linie A-A von Fig. 1 und verdeutlicht die Anordnung der beiden Katheterrohre zueinander.

Fig. 3 zeigt den Querschnitt durch den Doppelkatheter an einer anderen Stelle längs der Linie B-B von Fig. 1 und lässt erkennen, wie durch den eingeschobenen Stab 7 die seitlichen Löcher 4 während des Einführens in das Gefäss verschlossen sind. Dieser Querschnitt zeigt die Ausführungsform mit zwei seitlich angeordneten Löchern 4, die sich nicht gegenüber liegen, sondern einander angenähert sind. Die Fig. 2 und 3 zeigen die Ausführungsform des Doppelkatheters mit eiförmigem Gesamtquerschnitt und geben die Grössenverhältnisse der Innenrohre 1 und 3 nur angenähert wieder.

Fig. 4 zeigt die Ausführungsform mit aufgesetztem Ballon 8 am vorderen Ende 6 des Schienungskatheters 1. Die Zuführleitung 9 für das Aufblasmedium verläuft im Inneren längs der Wand des Schienungskatheters 1 und wird zusammen mit dem Nebenrohr 2 am hinteren Ende des Schienungskatheters 1 seitlich abgeführt. In der Figur ist die Abführung durch die Wand des Schienungskatheters gezeigt. Es ist aber auch möglich, die Zuführleitung 9 zunächst noch ein Stück innerhalb des Nebenrohres 2 zu führen und aus diesem dann später herauszuführen. Am Ende der Zuführleitung 9 ist ein sogenannter Luer-Konus 10 vorhanden zum Anschluss von Injektionsspritzen oder anderen Zuführvorrichtungen für das Aufblasmedium. Der aufblasbare Ballon hat eine Länge von 1,5-3 cm, vorzugsweise ist er 2 cm lang. Sein Abstand von der Abzweigung des kürzeren Katheterrohres 3 und der Abführstelle des Nebenrohres 2 vom Schienungskatheter 1 beträgt 15-18 cm, vorzugsweise 17 cm. Der Ballon kann innerhalb des Gefässes auf einen solchen Aussendurchmesser aufgeweitet werden, dass er an der Gefässinnenwand dicht anliegt. Eine Vergrösserung des Aussendurchmessers des Schienungskatheters 1 durch den Ballon um 5-10 mm ist dafür im allgemeinen ausreichend. Als Materialien für den Ballon können die für Ballonkatheter üblichen Materialien verwendet werden.

## Patentansprüche

1. Perfusionsdoppelkatheter, dessen einzelne fest miteinander verbundene Katheterrohre (1, 3) unterschiedliche Längen, unterschiedliche Querschnitte und unterschiedlich abgeschrägte vordere Öffnungen (5, 6) aufweisen und von dessen Katheterrohr (1) mit grösserem Aussendurchmesser ein Anschluss (2) seitlich abgeführt ist, dadurch gekennzeichnet, dass der Perfusionsdoppelkatheter aus einem Schienungskatheter (1) mit einem aufgesetzten kürzeren Katheterrohr (3), dessen hinteres Ende seitlich vom Schienungskatheter (1) abgeführt ist, besteht, wobei am Schienungskatheter (1) in Abstand von seinem hinteren Ende als seitlich abgeführter Anschluss ein Nebenrohr (2) und in einem Abstand von 40 bis 60 mm vom Mittelpunkt der Anschlussstelle des Nebenrohres (2) mindestens eine Öffnung (4) vorhanden sind.

2. Perfusionsdoppelkatheter nach Anspruch 1, dadurch gekennzeichnet, dass die Doppelkatheterrohre einen jeweils ovalen oder eiförmigen äusseren Querschnitt aufweisen und der Innendurchmesser des Schienungskatheters (1) grösser ist als der Innendurchmesser des kürzeren Katheterrohres (3).

3. Perfusionsdoppelkatheter nach Ansprüchen 1 oder 2, dadurch gekennzeichnet, dass die seitliche Abführung des kürzeren Katheterrohres (3) vom Schienungskatheter (1) in der Nähe der Anschlussstelle des Nebenrohres (2) angeordnet ist.

4. Perfusionsdoppelkatheter nach Ansprüchen 1 bis 3, dadurch gekennzeichnet, dass am vorderen Ende (5) des kürzeren Katheterrohres (3) zwei zueinander versetzte Öffnungen vorhanden sind.

5. Perfusionsdoppelkatheter nach Ansprüchen 1 bis 4, dadurch gekennzeichnet, dass der Abstand des Mittelpunktes der Öffnung (4) im Schienungskatheter (1) vom Mittelpunkt der Anschlussstelle des Nebenrohres (2) 50 mm beträgt.

6. Perfusionsdoppelkatheter nach Ansprüchen 1 bis 5, dadurch gekennzeichnet, dass die Öffnung (4) im Schienungskatheter (1) oval ausgebildet und etwa 10 bis 20 mm, vorzugsweise 16 mm, lang ist.

7. Perfusionsdoppelkatheter nach Ansprüchen 1 bis 6, dadurch gekennzeichnet, dass der Innendurchmesser des kürzeren Katheterrohres (3) 4 mm und der Innendurchmesser des Nebenrohres (2) 5 mm betragen.

8. Perfusionsdoppelkatheter nach Ansprüchen 1 bis 7, dadurch gekennzeichnet, dass im Schienungskatheter (1) ein geschlossenes Rohrstück oder ein Stab (7), dessen Aussendurchmesser etwas geringer ist als der Innendurchmesser des Schienungskatheters (1), auf einer solchen Länge angeordnet ist, dass die seitlichen Öffnungen (4) dadurch verschlossen sind, das Ende des Stabes (7) sich jedoch ausserhalb des hinteren Endes des Schienungskatheters (1) befindet.

9. Perfusionsdoppelkatheter nach Ansprüchen 1 bis 8, dadurch gekennzeichnet, dass er aus Siliconkautschuk besteht.

10. Perfusionsdoppelkatheter nach Ansprüchen 1 bis 9, dadurch gekennzeichnet, dass der Schienungskatheter (1) in geringem Abstand von seinem abgeschrägten vorderen Ende (6) einen aufgesetzten aufblasbaren Ballon (8) aufweist, dessen Zuführleitung (9) innerhalb des Schienungskatheters (1) verläuft und zusammen mit dem Nebenrohr (2) am hinteren Ende des Schienungskatheters (1) seitlich abgeführt ist und an ihrem Ende einen Luer-Konus (10) als Anschluss aufweist.

## Claims

1. A double perfusion catheter which has individual catheter tubes (1, 3) firmly connected to each other, of variable length, variable cross-section and variable slanted front orifices (5, 6), and from whose catheter tube (1) having a relatively large outer diameter, a connection (2) leads off at the side, characterised in that the double perfusion catheter comprises a splint catheter (1) with a relatively short catheter tube (3) positioned thereon, the rear end of which leads off at the side from the splint catheter (1), whereby on the splint catheter (1), at a spacing from the rear end thereof, there is a secondary tube (2) forming a connection leading off from the side, and there is at least one orifice (4) at a spacing of from 40 to 60 mm from the central point of the junction of the secondary tube (2).

2. A double perfusion catheter according to claim 1, characterised in that the double catheter tubes in each case have an oval or egg-shaped outer cross-section and the inner diameter of the splint catheter (1) is greater than the inner diameter of the shorter catheter tube (3).

3. A double perfusion catheter according to claims 1 or 2, characterised in that the side channel of the shorter catheter tube (3) from the splint catheter (1) is positioned in the vicinity of the junction of the secondary tube (2).

4. A double perfusion catheter according to claims 1 to 3, characterised in that two staggered orifices are present at the front end (5) of the shorter catheter tube (3).

5. A double perfusion catheter according to claims 1 to 4, characterised in that the spacing of the central point of the orifice (4) in the splint catheter (1) from the central point of the junction of the secondary tube (2) is 50 mm.

6. A double perfusion catheter according to claims 1 to 5, characterised in that the orifice (4) in the splint catheter (1) is oval-shaped and from about 10 to 20 mm, preferably 16 mm, in length.

7. A double perfusion catheter according to claims 1 to 6, characterised in that the inner diameter of the splint catheter (1) is from 8 to 16 mm, the inner diameter of the secondary tube (2) is 5 mm.

8. A double perfusion catheter according to claims 1 to 7, characterised in that in the rail catheter (1) a sealed tube section or a rod (7), the outer diameter of which is slightly smaller than the inner diameter of the splint catheter (1) is positioned on a length such that the side orifices (4) are sealed thereby, the end of the rod (7) being located, however, outside the rear end of the splint catheter (1).

9. A double perfusion catheter according to claims 1 to 8, characterised in that it consists of silicone rubber.

10. A double perfusion catheter according to claims 1 to 9, characterised in that the splint catheter (1) has an inflatable balloon (8) positioned thereon, at a small spacing from its slanted front end (6), the supply conduit (9) of which balloon runs in the splint catheter (1) and leads off from the side at the rear end of the splint catheter (1) together with the secondary tube (2), and has a Luer-cone (10) at its end as a connection.

## Revendications

1. Double cathéter à perfusion, dont les deux tubes cathéters (1, 3) liés l'un à l'autre de façon fixe ont des longueurs différentes, des sections transversales différentes et des orifices antérieurs (5, 6) biseautés de façon différente et dont le tube cathéter (1) de plus grand diamètre extérieur comporte un raccordement (2) latéral, caractérisé en ce que le double cathéter à perfusion est constitué d'un cathéter de maintien (1) et d'un tube cathéter plus court (3) placé dessus et dont l'extrémité postérieure s'écarte latéralement du cathéter de maintien (1), le cathéter de maintien (1) comportant:

— à une certaine distance de son extrémité postérieure, un tube de dérivation (2) raccordé latéralement et

— à une distance de 40 à 60 mm du centre de la région de raccordement du tube de dérivation (2), au moins un orifice (4).

2. Double cathéter à perfusion selon la revendication 1, caractérisé en ce que le cathéter à double tube présente une section transversale extérieure ovale ou en forme d'œuf et en ce que le diamètre intérieur du cathéter de maintien (1) est plus grand que le diamètre intérieur du tube cathéter court (3).

3. Double cathéter à perfusion selon la revendication 1 ou 2, caractérisé en ce que la déviation latérale du tube cathéter court (3) par rapport au cathéter de maintien (1) est située à proximité de la région de raccordement du tube de dérivation (2).

4. Double cathéter à perfusion selon l'une quelconque des revendications 1 à 3, caractérisé en ce qu'à l'extrémité antérieure (5) du tube cathéter court (3) se trouvent deux orifices décalés l'un par rapport à l'autre.

5. Double cathéter à perfusion selon l'une quelconque des revendications 1 à 4, caractérisé en ce que la distance entre le centre de l'orifice (4) dans le cathéter de maintien (1) et le centre de la région de raccordement du tube de dérivation (2) est de 50 mm.

6. Double cathéter à perfusion selon l'une quelconque des revendications 1 à 5, caractérisé en ce que l'orifice (4) dans le cathéter de maintien (1) a une forme ovale et une longueur d'environ 10 à 20 mm, de préférence de 16 mm.

7. Double cathéter à perfusion selon l'une quelconque des revendications 1 à 6, caractérisé en ce que le diamètre intérieur du cathéter de maintien (1) est de 8 à 16 mm, le diamètre intérieur du tube cathéter court (3) est de 4 mm et le diamètre intérieur du tube de dérivation (2) est de 5 mm.

8. Double cathéter à perfusion selon l'une quelconque des revendications 1 à 7, caractérisé en ce que dans le cathéter de maintien (1) est disposé un morceau de tuyau fermé ou une baguette (7), dont le diamètre extérieur est un peu plus petit que le diamètre intérieur du cathéter de maintien (1), sur une longueur telle que les orifices latéraux (4) sont obturés et que cependant l'extrémité de la baguette (7) se trouve à l'extérieur de l'extrémité postérieure du cathéter de maintien (1).

9. Double cathéter à perfusion selon l'une quelconque des revendications 1 à 8, caractérisé en ce qu'il est constitué de caoutchouc siliconé.

10. Double cathéter à perfusion selon l'une quelconque des revendications 1 à 9, caractérisé en ce que sur le cathéter de maintien (1), à une courte distance de son extrémité antérieure biseautée (6), est placé un ballon gonflable (8), dont la conduite d'amenée (9) passe à l'intérieur du cathéter de maintien (1), dévie latéralement avec le tube de dérivation (2) à l'extrémité postérieure du cathéter de maintien (1) et présente à son extrémité un cône de Luer (10) comme pièce de raccordement.

Fig. 1

Fig. 2

Fig. 3

Fig. 4

0 093 887